# EUROPEAN PATENT APPLICATION

(11) **EP 2 586 305 A1**
(43) Date of publication of application: **01.05.2013**
(21) Application number: 12194393.0
(22) Date of filing: 12.09.2008
(51) Int. Cl.: A01N 1/02, A61K 38/18, A61P 9/04, C07K 14/475

(54) **Use of neuregulin for organ preservation**

(30) Priority: 12.09.2007 US 993566 P
(62) Divisional of application: 08800603.6
(71) Applicant: Zensun (Shanghai) Science and Technology Limited, Shanghai 201203 (CN)
(72) Inventor: Zhou, Mingdong, Shanghai 201203 (CN)
(74) Representative: Jones Day

(57) **Abstract**

The invention relates to a composition for perfusion, preservation or reperfusion of an organ for transplantation, comprising a preservation solution and an effective amount of neuregulin. The invention further relates to the use of neuregulin for preserving an organ for transplantation, increasing the relaxation speed of heart and treating chronic heart failure in a subject.

SEQUENCE LISTING

<110> Zensun (Shanghai) Science and Technology Limited

<120> USE OF NEUREGULIN FOR ORGAN PRESERVATION

<130> 102436PCEPT1

<140>
<141> 2008-09-12

<150> US 60/993,566
<151> 2007-9-12

<160> 2

<170> FastSEQ for Windows Version 4.0

<210> 1
<211> 61
<212> PRT
<213> Homo sapiens

<400> 1
Ser His Leu Val Lys Cys Ala Glu Lys Glu Lys Thr Phe Cys Val Asn
 1               5                   10                  15
Gly Gly Glu Cys Phe Met Val Lys Asp Leu Ser Asn Pro Ser Arg Tyr
            20                  25                  30
Leu Cys Lys Cys Pro Asn Glu Phe Thr Gly Asp Arg Cys Gln Asn Tyr
        35                  40                  45
Val Met Ala Ser Phe Tyr Lys Ala Glu Glu Leu Tyr Gln
    50                  55                  60

<210> 2
<211> 183
<212> DNA
<213> Homo sapiens

<400> 2
agccatcttg taaaatgtgc ggagaaggag aaaactttct gtgtgaatgg aggggagtgc 60
ttcatggtga aagacctttc aaacccctcg agatacttgt gcaagtgccc aaatgagttt 120
actggtgatc gctgccaaaa ctacgtaatg gccagcttct acaaggcgga ggagctgtac 180
cag                                                               183

## Description

This application claims the benefit of priority of U.S. provisional application no. 60/993,566, filed September 12, 2007, the contents of which are hereby incorporated by reference in their entireties.

### 1. FIELD OF THE INVENTION

The present invention relates to compositions and methods for preservation, perfusion or reperfusion of an organ, such as heart, for transplantation.

### 2. BACKGROUND OF THE INVENTION

Successful organ transplantation is often limited due to ischemic/reperfusion injury. Isolated human hearts deprived of oxygen for more than four hours progressively loose vigor and often do not survive in recipient hosts. Other organs such as the kidney, liver, pancreas and lung are also subject to tissue and cellular damage when removed from their hosts prior to transplantation. This damage is due to hypoxic conditions and a lack of circulation, which normally delivers physiological concentrations of oxygen and nutrients, and removes toxic compounds produced by an organ's cells. Organ transplants have a higher frequency of success when performed immediately after excision from their hosts.

Recent advances have increased the rate of successful organ transplants and organ surgery, such as coronary bypass surgery. The first includes organ preservation and organ perfusion solutions. The second is improved methods and devices for the delivery of organ perfusion solutions to an organ.

Short-term myocardiac preservation is currently provided by cold storage after cardioplegic arrest. A variety of processes exist however differing by the composition of the solution used, the preservation temperature and the administration protocol. Different solutions for arresting and preserving the heart have been developed to protect the myocardium in cardiac surgery. Examples of these solutions include Krebs-He+seleit solution, UW solution, St. Thomas II solution, Collins solution and Stanford solution. (See, e.g., U.S. Pat. Nos. 4,798,824 and 4,938,961; Southard and Belzer, Ann. Rev. Med. 46:235-247 (1995); and Donnelly and Djuric, Am. J. Hosp. Pharm. 48:2444-2460 (1991)). Nevertheless, organ rejections still remains due to deterioration in the condition of the transplanted organ between the time of removal and the restoration of blood flow in the recipient.

It has recently been found that neuregulin (hereinafter referred to as "NRG") can cause cardiomyocyte growth and/or differentiation. NRGs comprise a family of structurally related growth and differentiation factors that include NRG1, NRG2, NRG3 and NRG4 and isoforms thereof. For example, over 15 distinct isoforms of NRG1 have been identified and divided into two large groups, known as α- and β- types, on the basis of differences in the sequence of their essential epidermal growth factor (EGF)-like domains.

NRGs bind to the EGF receptor family, which comprises EGFR, ErbB2, ErbB3 and ErbB4, each of which plays an important role in multiple cellular functions, including cell growth, differentiation and survival. They are protein tyrosine kinase receptors, consisting of an extracellular ligand-binding domain, transmembrane domain and cytoplasmic tyrosine kinase domain. After NRG binds to the extracellular domain of ErbB3 or ErbB4, it induces a conformational change that leads to heterodimer formation between ErbB3, ErbB4 and ErbB2 or homodimer formation between ErbB4 itself, which results in phosphorylation of the receptors' C-terminal domain inside the cell membrane. The phosphorylated intracellular domain then binds additional signal proteins inside the cell, activating the corresponding downstream AKT or ERK signaling pathway, and inducing a series of cell reactions, such as stimulation or depression of cell proliferation, cell differentiation, cell apoptosis, cell migration or cell adhesion. Among these receptors, mainly ErbB2 and ErbB4 are expressed in the heart.

It has been shown that the EGF-like domains of NRG1, ranging in size from 50 to 64-amino acids, are sufficient to bind to and activate these receptors. Previous studies have shown that neuregulin-1β (NRG-1β) can bind directly to ErbB3 and ErbB4 with high affinity. The orphan receptor, ErbB2, can form heterodimer with ErbB3 or ErbB4 with higher affinity than ErbB3 or ErbB4 homodimers. Research in neural development has indicated that the formation of the sympathetic nervous system requires an intact NRG-1β, ErbB2 and ErbB3 signaling system. Targeted disruption of the NRG-1β or ErbB2 or ErbB4 led to embryonic lethality due to cardiac development defects. Recent studies also highlighted the roles of NRG-1β, ErbB2 and ErbB4 in the cardiovascular development as well as in the maintenance of adult normal heart function. NRG-1β has been shown to enhance sarcomere organization in adult cardiomyocytes. The short-term administration of a recombinant NRG-1β EGF-like domain significantly improves or protects against deterioration in myocardial performance in three distinct animal models of heart failure. More importantly, NRG-1β significantly prolongs survival of heart failure animals.

Therefore, there remains a need for a composition that can extend the preservation time of an organ for transplantation and protect the organ from reperfusion injury after ischemia, so that the organ can resume proper function after restoration of blood flow. The present invention provides methods and compositions comprising neuregulin for preservation, perfusion or reperfusion of an organ.

### 3. SUMMARY OF THE INVENTION

The present invention provides compositions for preservation, perfusion, and/or reperfusion of an organ, such as the heart, comprising a preservation solution and neuregulin. The preservation solution and neuregulin are described in detail below. The compositions protects organ tissues and cells from damage while the organ is isolated from the circulatory system or is experiencing decreased blood flow (ischemia). It has been found that neuregulin can exert protective effects in organs undergoing ischemia/reperfusion.

The compositions of the present invention can be used according to the judgment of one of skill in the art. For example, the compositions can be for perfusing an organ for transplantation by being pumped into the vasculature of the organ to protect the organ tissues and cells. Alternatively, the compositions can also be used for preserving an organ for transplantation by serving as a bathing solution into which the organ is submerged. Preferably, the organ is perfused with and submerged in the present compositions. Further, the present compositions also can be used a reperfusion solution upon restoration of blood flow to the organ after ischemia.

The present invention provides methods of using the compositions of the present invention. These include methods for preserving an organ for transplantation, for protecting an ischemic organ from damage, for attenuating organ dysfunction after ischemia, and for protecting an organ from damage when isolated from the circulatory system.

The present invention also provides methods for treating chronic heart failure comprising administering the subject in need thereof an effective amount of neuregulin to the subject for six hours.

### 4. BRIEF DESCRIPTION OF THE DRAWING

Figure 1 shows the time needed for cardiomyocytes to relax to half of the maximum length. Con and MI stand for myocytes from sham operated rats and myocardial infarcted rats respetively. The second column for MI myocytes were treated with NRG, and the time unit is mili-second(ms). The cells measured for Con, MI, MI with NRG were 68, 97 and 100 separately from different rats.

Figure 2 shows AKT phosphorylation in different heart tissue after stored at various conditions for 6 hours. The first 3 lanes were for heart stored in Celsior solution alone, 4-6 lane for heart stored in Celsior solution containing neuregulin, 7-9 lane for heart preincubated with wortmannin before storage in Celsior solution containing neuregulin. WM and P-AKT stand for wortmannin and phosphorylated AKT respectively.

Figure 3 shows the average cardiac output for patients with stable chronic heart failure after continuous infusion of neuregulin in 24 hours.

Figure 4 shows the average systemic vascular resistance (SVR) for patients with stable chronic heart failure after continuous infusion of neuregulin in 24 hours.

### 5. DETAILED DESCRIPTION OF THE INVENTION

### 5.1 Definitions

Unless defined otherwise, all technical and scientific terms used herein have the same meaning as is commonly understood by one of ordinary skill in the art to which this invention belongs. All patents, applications, published applications and other publications referred to herein are incorporated by reference in their entirety. If a definition set forth in this section is contrary to or otherwise inconsistent with a definition set forth in the patents, applications, published applications and other publications that are herein incorporated by reference, the definition set forth in this section prevails over the definition that is incorporated herein by reference.

As used herein, the singular forms "a", "an", and "the" mean "at least one" or "one or more" unless the context clearly dictates otherwise.

The term "about" or "approximately" means within 20%, preferably within 10%, and more preferably within 5% (or 1% or less) of a given value or range.

As used herein, "administer" or "administration" refers to the act of injecting or otherwise physically delivering a substance as it exists outside the body *(e.g.,* a composition provided herein) into a patient, such as by mucosal, intradermal, intravenous, intramuscular delivery and/or any other method of physical delivery described herein or known in the art. When a disease, or a symptom thereof, is being treated, administration of the substance typically occurs after the onset of the disease or symptoms thereof. When a disease, or symptoms thereof, are being prevented, administration of the substance typically occurs before the onset of the disease or symptoms thereof.

As used herein, "neuregulin" or "NRG" used in the present invention refers to proteins or peptides that can bind and activate ErbB2, ErbB3, ErbB4 or combinations thereof, including but not limited to all neuregulin isoforms, neuregulin EGF domain alone, polypeptides comprising neuregulin EGF-like domain, neuregulin mutants or derivatives, and any kind of neuregulin-like gene products that also activate the above receptors as described in detail below. In preferred embodiments, neuregulin used in the present invention binds to and activates ErbB2/ErbB4 or ErbB2/ErbB3 heterodimers. Neuregulin also includes NRG-1, NRG-2, NRG-3, and NRG-4 proteins, peptides, fragments and compounds that mimic the activities of neuregulin. Neuregulin used in the present invention can activate the above ErbB receptors and modulate their biological reactions, *e.g.,* stimulate breast cancer cell differentiation and milk protein secretion; induce the differentiation of neural crest cell into Schwann cell; stimulate acetylcholine receptor synthesis in skeletal muscle cell; and/or improve cardiocyte differntiation, survival and DNA synthesis. Neuregulin also includes those variants with conservative amino acid substitutions that do not substantially alter their biological activity. Suitable conservative substitutions of amino acids are known to those of skill in this art and may be made generally without altering the biological activity of the resulting molecule. Those of skill in this art recognize that, in general, single amino acid substitutions in non-essential regions of a polypeptide do not substantially alter biological activity (see, e.g., Watson et al. Molecular Biology of the Gene, 4th Edition, 1987, The Bejacmin/Cummings Pub. co., p.224).

Neuregulin protein encompasses neuregulin protein and peptide. Neuregulin nucleic acid encompasses neuregulin nucleic acid and neuregulin oligonucleotide.

As used herein, "epidermal growth factor-like domain" or "EGF-like domain" refers to a polypeptide motif encoded by the neuregulin gene that binds to and activates ErbB2, ErbB3, ErbB4, or combinations thereof, and bears a structural similarity to the EGF receptor-binding domain as disclosed in WO 00/64400, Holmes et al., Science, 256:1205-1210 (1992); U.S. Patent Nos. 5,530,109 and 5,716,930; Hijazi et al., Int. J. Oncol., 13:1061-1067 (1998); Chang et al., Nature, 387:509-512 (1997); Carraway et al., Nature, 387:512-516 (1997); Higashiyama et al., J. Biochem., 122:675-680 (1997); and WO 97/09425, the contents of which are all incorporated herein by reference. In certain embodiments, EGF-like domain binds to and activates ErbB2/ErbB4 or ErbB2/ErbB3 heterodimers. In certain embodiments, EGF-like domain comprises the amino acid sequence of the receptor binding domain of NRG-1. In some embodiments, EGF-like domain comprises the amino acid sequence corresponding to amino acid residues 177-226, 177-237, or 177-240 of NRG-1. In certain embodiments, EGF-like domain comprises the amino acid sequence of the receptor binding domain of NRG-2. In certain embodiments, EGF-like domain comprises the amino acid sequence of the receptor binding domain of NRG-3. In certain embodiments, EGF-like domain comprises the amino acid sequence of the receptor binding domain of NRG-4. In certain embodiments, EGF-like domain comprises the amino acid sequence of Ala Glu Lys Glu Lys Thr Phe Cys Val Asn Gly Gly Glu Cys Phe Met Val Lys Asp Leu Ser Asn Pro, as described in U.S. Pat. No. 5,834,229.

As used herein, "ejection fraction" or "EF" means the portion of blood that is pumped out of a filled ventricle as the result of a heartbeat. It may be defined by the following formula: (LV diastolic volume - LV systolic volume) / LV diastolic volume.

As used herein, "heart failure" means an abnormality of cardiac function where the heart does not pump blood at the rate needed for the requirements of metabolizing tissues. Heart failure includes a wide range of disease states such as congestive heart failure, myocardial infarction, tachyarrhythmia, familial hypertrophic cardiomyopathy, ischemic heart disease, idiopathic dilated cardiomyopathy, myocarditis and the like. The heart failure can be caused by any number of factors, including, without limitation, ischemic, congenital, rheumatic, or idiopathic forms. Chronic cardiac hypertrophy is a significantly diseased state which is a precursor to congestive heart failure and cardiac arrest.

As used herein, "protein" is synonymous with "polypeptide" or "peptide" unless the context clearly dictates otherwise.

As used herein, the terms 'subject' and 'patient' are used interchangeably. As used herein, a subject is preferably a mammal, such as a non-primate *(e.g.,* cows, pigs, horses, cats, dogs, rats, *etc.)* or a primate *(e.g.,* monkey and human), most preferably a human.

As used herein, "activity unit" or "1U" means the quantity of standard product that can induce 50% maximal reaction. In other words, to determine the activity unit for a given active agent, the EC50 must be measured. For example, if the EC50 for a batch of product was 0.067 µg/ml then that would be one unit. Further, if 1 µg of that product is being used then 14.93U (1 / 0.067) is being used. The EC50 can be determined by any method known in the art, including the method employed by the inventors in the Examples below. This determination of the activity unit is important for quality control of genetically engineered products and clinically used drugs, permits product from different pharmaceuticals and/or different batch numbers to be quantified with uniform criteria.

In certain embodiments, unit of neuregulin is determined by measuring the activity of neuregulin through kinase receptor activation enzyme-linked immunosorbant assay (KIRA-ELISA) as described in detail in Example 6 below and in WO03/099300, and Sadick et al., 1996, Analytical Biochemistry, 235:207-14, the contents of which are incorporated by reference in their entireties. Briefly, the assay measures neuregulin induced ErbB2 activation and phosphorylation on the adherent breast carcinoma cell line, MCF-7. Membrane proteins are solubilized via Triton X-100 lysis and the receptor is captured in ELISA wells coated with ErbB2-specific antibodies (e.g., 114) with no cross-reaction to ErbB3 or ErbB4. The degree of receptor phosphorylation is then quantified by antiphosphotyrosine ELISA.

### 5.2 Compositions for Preservation, Perfusion or Reperfusion of Heart for Transplantation and Uses Thereof

The present invention provides compositions for the preservation, perfusion, and/or reperfusion of an organ, such as the heart. The compositions comprise a preservation solution and an effective amount of neuregulin. It has been found that neuregulin can protect an organ such as heart, from oxidative damage, ischemia and repurfusion injury while the organ is isolated from the normal circulation or receives inadequate arterial flow

Any neuregulin known in the art can be used in the present composition. Neuregulin is described in detail below. The compositions comprise an effective amount of neuregulin to protect the organ from damage due to tissue anoxia, ischemia or reperfusion injury. In some embodiments, neuregulin is present in an amount sufficient to active AKT signaling pathway. In some embodiments, neuregulin is at the concentration at least about 0.01 nM, about 0.1 nM, about 1 nM, about 10 nM, about 100 nM, about 1000 nM, about 10 µM, about 100 µM, or about 1 000 µM. In some embodiments, neuregulin is at the concentration of about 14 nM. In some embodiments, neuregulin is at the concentration of about 0.01-1000 nM, about 0.1-100 nM, about 0.1-50 nM, about 0.5 -25 nM, or about 10-20 nM. In some embodiments, neuregulin is at the concentration of about 14 nM.

The preservation solutions in the present compositions can be any preservation solutions known in the art for organ perfusion, preservation or reperfusion. Exemplary preservation solutions include but are not limited to Celsior solution, Krebs-Henseleit solution, normal saline solution, University of Wisconsin solution, St. Thomas II solution, Collins solution, Stanford solution, *etc.*

The compositions is preferably maintained at physiological pH of about 7.0-7.5, more preferably about 7.2-7.4.

The compositions may also contain one or more of agents known in the art for protecting tissue from damage during transplantation or from acute ischemia due to injury or surgery. Such agents include but are not limited to Protein kinase C inhibitors, bupivacaine, levo-bupivacaine, etidocaine, ropivacaine, or tetracaine, such as described in detail in U.S. Pat. App. Pub. Nos. 20070148628, 20060166182.

The present invention provides methods of using the compositions of the present invention. These include methods for preserving an organ for transplantation, for protecting an ischemic organ from damage, for attenuating organ dysfunction after ischemia, and for protecting an organ from damage when isolated from the circulatory system.

The compositions of the present invention can be used during all phases of an organ, especially the heart, transplant, including, but are not limited to, 1) isolating of the organ from the donor; 2) preserving the organ; and 3) re-implanting the organ in the recipient.

The present invention provides methods for preserving an organ, such as heart, for transplant, comprising contacting the organ with an effective amount of neuregulin. The tissue can be contacted with neuregulin prior to removal from the subject, and/or after organ removal from the subject and/or during removal of the organ from the subject.

The compositions of the present invention can be prepared by any method known in the art. For example, the compositions can be prepared by 1) dissolving and diluting neuregulin and the different constituents in distilled water; 2) adjusting the pH to the desired value, such as about 7.2-7.4, e.g. with NaOH; and 3) sterilizing the solution, e.g., by filtering with a 0.2 µM filter. The sterilized solution is then kept isolated from contaminants in the environment.

### 5.2.1 Neuregulin

Any NRG (e.g., NRG-1, NRG-2, NRG-3 and NRG-4 and isoforms thereof) protein, peptide or fragment can be used in the present invention.

Neuregulin or NRG refers to proteins or peptides that can bind and activate ErbB2, ErbB3, ErbB4 or combinations thereof, including but not limited to all neuregulin isoforms, neuregulin EGF domain alone, polypeptides comprising neuregulin EGF-like domain, neuregulin mutants or derivatives, and any kind of neuregulin-like gene products that also activate the above receptors as described in detail below. In preferred embodiments, neuregulin used in the present invention binds to and activate ErbB2/ErbB4 or ErbB2/ErbB3 heterodimers. Neuregulin used in the present invention can activate the above ErbB receptors and modulate their biological reactions, e.g., stimulate breast cancer cell differentiation and milk protein secretion; induce the differentiation of neural crest cell into Schwann cell; stimulate acetylcholine receptor synthesis in skeletal muscle cell; and/or improve cardiocyte differentiation, survival and DNA synthesis. Assays for measuring the receptor binding activity are known in the art. For example, cells transfected with ErbB-2 and ErbB-4 receptor can be used. After receptor expressing cells are incubated with excess amount of radiolabeled neuregulin, the cells are pelleted and the solution containing unbound radiolabeled neuregulin is removed before unlabeled neuregulin solution is added to compete with radiolabeled neuregulin. EC50 is measured by methods known in the art. EC50 is the concentration of ligands which can compete 50% of bound radiolabeled ligands off the receptor complex. The higher the EC50 value is, the lower the receptor binding affinity is.

Neuregulin used in the present invention includes any neuregulin and isoforms thereof known in the art, including but not limited to all isoforms of neuregulin-1 ("NRG-1"), neuregulin-1 ("NRG-2"), neuregulin-1 ("NRG-3") and neuregulin-4 ("NRG-43"). NRG-1 is described, for example, in U.S. Pat. Nos. 5,530,109, 5,716,930, and 7,037,888; Lemke, Mol. Cell. Neurosci. 1996, 7:247-262; Peles and Yarden, 1993, BioEssays 15:815-824, 1993; Peles et al., 1992, Cell 69, 205-216; Wen et al., 1992, Cell 69, 559-572, 1992, Holmes et al., 1992, Science 256:1205-1210, Falls et al., 1993, Cell 72:801-815, Marchionni et al. 1993, Nature 362:312-8, the contents of which are incorported by reference in their entireties. NRG-2 is described, for example, in Chang et al., 1997, Nature 387:509-512; Carraway et al., 1997, Nature 387:512-516; Higashiyama et al., 1997, J. Biochem. 122:675-680, Busfield et al., 1997, Mol. Cell. Biol. 17:4007-4014 and International Pat. Pub. No. WO 97/09425), the contents of which are incorported by reference in their entireties. NRG-3 is described, for example, in Hijazi et al., 1998, Int. J Oncol. 13:1061-1067, the contents of which are incorported by reference in their entireties. NRG-4 is described, for example, in Harari et al., 1999 Oncogene. 18:2681-89, the contents of which are incorported by reference in their entireties.

Neuregulin used in the present invention includes neuregulin mutants or derivatives that comprise one or more amino acid substitutions, deletions, and/or additions that are not present in the naturally occurring neuregulin. Preferably, the number of amino acids substituted, deleted, or added is 1, 2, 3, 4, 5, 6, 7, 8. 9, or 10 amino acids. In one embodiment, such a derivative contains one or more amino acid deletions, substitutions, or additions at the amino and/or carboxy terminal end of the peptide. In another embodiment, such a derivative contains one or more amino acid deletions, substitutions, or additions at any residue within the length of the peptide.

In certain embodiments, the amino acid substitutions may be conservative or non-conservative amino acid substitutions. Conservative amino acid substitutions are made on the basis of similarity in polarity, charge, solubility, hydrophobicity, hydrophilicity, and/or the amphipathic nature of the amino acid residues involved. For example, nonpolar (hydrophobic) amino acids include alanine, leucine, isoleucine, valine, proline, phenylalanine, tryptophan, and methionine; polar neutral amino acids include glycine, serine, threonine, cysteine, tyrosine, asparagine, and glutamine; positively charged (basic) amino acids include arginine, lysine, and histidine; and negatively charged (acidic) amino acids include aspartic acid and glutamic acid. In addition, glycine and proline are residues that can influence chain orientation. Non-conservative substitutions will entail exchanging a member of one of these classes for another class.

In certain embodiments, neuregulin used in the present invention is a neuregulin derivative with conservative amino acid substitutions that do not substantially alter their biological activity. Suitable conservative substitutions of amino acids are known to those of skill in this art and may be made generally without altering the biological activity of the resulting molecule. Those of skill in this art recognize that, in general, single amino acid substitutions in non-essential regions of a polypeptide do not substantially alter biological activity *(see, e.g.,* Watson et al. Molecular Biology of the Gene, 4th Edition, 1987, The Bejacmin/Cummings Pub. co., p.224).

In certain embodiments, neuregulin used in the present invention includes neuregulin mutants or derivatives having an amino acid substitution with a non-classical amino acid or chemical amino acid analog. Non-classical amino acids include, but are not limited to, the D-isomers of the common amino acids, a -amino isobutyric acid, 4-aminobutyric acid, Abu, 2-amino butyric acid, γ-Abu, ε-Ahx, 6-amino hexanoic acid, Aib, 2-amino isobutyric acid, 3-amino propionic acid, ornithine, norleucine, norvaline, hydroxyproline, sarcosine, citrulline, cysteic acid, t-butylglycine, t-butylalanine, phenylglycine, cyclohexylalanine, β-alanine, fluoro-amino acids, designer amino acids such as β-methyl amino acids, Cα-methyl amino acids, Nα-methyl amino acids, and amino acid analogs in general.

Neuregulin used in the present invention includes neuregulin homologue, that is, a polypeptide that exhibits an amino acid sequence homology and/or structural resemblance to neuregulin, or to one of the interacting domains of neuregulin such that it is capable of bind and activate ErbB2/ErbB4 or ErbB2/ErbB3 heterodimers protein kinases. Typically, a protein homologue of a native protein may have an amino acid sequence that is at least 50%, preferably at least 75%, more preferably at least 80%, 85%, 86%, 87%, 88% or 89%, even more preferably at least 90%, 91%, 92%, 93% or 94%, and most preferably 95%, 96%, 97%, 98% or 99% identical to the native protein.

Percent homology in this context means the percentage of amino acid residues in the candidate sequence that are identical *(i.e.,* the amino acid residues at a given position in the alignment are the same residue) or similar (*i.e*., the amino acid substitution at a given position in the alignment is a conservative substitution, as discussed above), to the corresponding amino acid residue in the peptide after aligning the sequences and introducing gaps, if necessary, to achieve the maximum percent sequence homology. In certain embodiments, neuregulin homologue is characterized by its percent sequence identity or percent sequence similarity with the naturally occurring neuregulin sequence. Sequence homology, including percentages of sequence identity and similarity, are determined using sequence alignment techniques well-known in the art, preferably computer algorithms designed for this purpose, using the default parameters of said computer algorithms or the software packages containing them.

Nonlimiting examples of computer algorithms and software packages incorporating such algorithms include the following. The BLAST family of programs exemplify a preferred, non-limiting example of a mathematical algorithm utilized for the comparison of two sequences (*e.g*., Karlin & Altschul, 1990, Proc. Natl. Acad. Sci. USA 87:2264-2268 (modified as in Karlin & Altschul, 1993, Proc. Natl. Acad. Sci. USA 90:5873-5877), Altschul et al., 1990, J. Mol. Biol. 215:403-410, (describing NBLAST and XBLAST), Altschul et al., 1997, Nucleic Acids Res. 25:3389-3402 (describing Gapped BLAST, and PSI-Blast). Another preferred example is the algorithm of Myers and Miller (1988 CABIOS 4:11-17) which is incorporated into the ALIGN program (version 2.0) and is available as part of the GCG sequence alignment software package. Also preferred is the FASTA program (Pearson W.R. and Lipman D.J., Proc. Nat. Acad. Sci. USA, 85:2444-2448, 1988), available as part of the Wisconsin Sequence Analysis Package. Additional examples include BESTFIT, which uses the "local homology" algorithm of Smith and Waterman (Advances in Applied Mathematics, 2:482-489, 1981) to find best single region of similarity between two sequences, and which is preferable where the two sequences being compared are dissimilar in length; and GAP, which aligns two sequences by finding a "maximum similarity" according to the algorithm of Neddleman and Wunsch (J. Mol. Biol. 48:443-354, 1970), and is preferable where the two sequences are approximately the same length and an alignment is expected over the entire length.

Examples of homologues may be the ortholog proteins of other species including animals, plants, yeast, bacteria, and the like. Homologues may also be selected by, e.g., mutagenesis in a native protein. For example, homologues may be identified by site-specific mutagenesis in combination with assays for detecting protein-protein interactions. Additional methods, e.g., protein affinity chromatography, affinity blotting, *in vitro* binding assays, and the like, will be apparent to skilled artisans apprised of the present invention.

For the purpose of comparing two different nucleic acid or polypeptide sequences, one sequence (test sequence) may be described to be a specific "percent identical to" another sequence (reference sequence) in the present disclosure. In this respect, when the length of the test sequence is less than 90% of the length of the reference sequence, the percentage identity is determined by the algorithm of Myers and Miller, Bull. Math. Biol., 51:5-37 (1989) and Myers and Miller, Comput. Appl. Biosci., 4(1):11-17 (1988). Specifically, the identity is determined by the ALIGN program. The default parameters can be used.

Where the length of the test sequence is at least 90% of the length of the reference sequence, the percentage identity is determined by the algorithm of Karlin and Altschul, Proc. Natl. Acad Sci. USA, 90:5873-77 (1993), which is incorporated into various BLAST programs. Specifically, the percentage identity is determined by the "BLAST 2 Sequences" tool. See Tatusova and Madden, FEMS Microbiol. Lett., 174(2):247-250 (1999). For pairwise DNA-DNA comparison, the BLASTN 2.1.2 program is used with default parameters (Match: 1; Mismatch: - 2; Open gap: 5 penalties; extension gap: 2 penalties; gap x_dropoff: 50; expect: 10; and word size: 11, with filter). For pairwise protein-protein sequence comparison, the BLASTP 2.1.2 program is employed using default parameters (Matrix: BLOSUM62; gap open: 11; gap extension: 1; x_dropoff: 15; expect: 10.0; and wordsize: 3, with filter).

Neuregulin used in the present invention also include neuregulin EGF domain alone, polypeptides comprising neuregulin EGF domain or neuregulin-like gene products that mimic the activities of neuregulin and binds and activates ErbB2, ErbB3, ErbB4 or combinations thereof. As used herein, "epidermal growth factor-like domain" or "EGF-like domain" refers to a polypeptide motif encoded by the neuregulin gene that binds to and activates ErbB2, ErbB3, ErbB4, or combinations thereof, and bears a structural similarity to the EGF receptor-binding domain as disclosed in WO 00/64400, Holmes et al., Science, 256:1205-1210 (1992); U.S. Patent Nos. 5,530,109 and 5,716,930; Hijazi et al., Int. J. Oncol., 13:1061-1067 (1998); Chang et al., Nature, 387:509-512 (1997); Carraway et al., Nature, 387:512-516 (1997); Higashiyama et al., J. Biochem., 122:675-680 (1997); and WO 97/09425, the contents of which are all incorporated herein by reference.

In certain embodiments, neuregulin used in the present invention comprises the EGF-like domain encoded by NRG-1. In some embodiments, EGF-like domain comprises the amino acid sequence of the receptor binding domain of NRG-1. In some embodiments, EGF-like domain comprises the amino acid sequence corresponding to amino acid residues 177-226, 177-237, or 177-240 of NRG-1.

In preferred embodiments, neuregulin used in the present invention comprises the amino acid sequence of : Ser His Leu Val Lys Cys Ala Glu Lys Glu Lys Thr Phe Cys Val Asn Gly Gly Glu Cys Phe Met Val Lys Asp Leu Ser Asn Pro Ser Arg Tyr Leu Cys Lys Cys Pro Asn Glu Phe Thr Gly Asp Arg Cys Gln Asn Tyr Val Met Ala Ser Phe Tyr Lys Ala Glu Glu Leu Tyr Gln (SEQ ID NO:1), which corresponds to amino acids 177-237 of human NRG-1. The human nucleic acid sequence encoding the fragment is: agccatcttg taaaatgtgc ggagaaggag aaaactttct gtgtgaatgg aggggagtgc ttcatggtga aagacctttc aaacccctcg agatacttgt gcaagtgccc aaatgagttt actggtgatc gctgccaaaa ctacgtaatg gcgagcttct acaaggcgga ggagctgtac cag (SEQ ID NO:2).

In certain embodiments, neuregulin used in the present invention comprises the EGF-like domain encoded by NRG-2. In certain embodiments, neuregulin used in the present invention comprises the EGF-like domain encoded by NRG-3. In certain embodiments, neuregulin used in the present invention comprises the EGF-like domain encoded by NRG-4. In certain embodiments, neuregulin used in the present invention comprises the amino acid sequence of Ala Glu Lys Glu Lys Thr Phe Cys Val Asn Gly Gly Glu Cys Phe Met Val Lys Asp Leu Ser Asn Pro, as described in U.S. Pat. No. 5,834,229.

### 5.3 Methods ForTreating Chronic Heart Failure

The present invention also provides methods for treating hear failure in a subject in need thereof comprising adiminstering to the subject an effective amount of neuregulin. In some embodiments, neuregulin is adminstered for about six hours at 0.2 µg/ml/kg/hour. The amount of neuregulin used will vary with the nature and severity of the disease or condition, and the route by which the active ingredient is administered. The frequency and dosage will also vary according to factors specific for each patient depending on the specific therapy (e.g., therapeutic or prophylactic agents) administered, the severity of the disorder, disease, or condition, the route of administration, as well as age, body, weight, response, and the past medical history of the patient. Effective doses may be extrapolated from dose-response curves derived from in vitro or animal model test systems.
Exemplary doses of neuregulin include milligram or microgram amounts of neuregulin per kilogram of subject or sample weight *(e.g.,* about I microgram per kilogram to about 500 milligrams per kilogram, about 100 micrograms per kilogram to about 5 milligrams per kilogram, or about 1 microgram per kilogram to about 50 micrograms per kilogram). For extened release of neuregulin used in the invention, the dosage administered to a patient is typically 0.001 mg/kg to 15 mg/kg of the patient's body weight, based on weight of the active peptide. Preferably, the dosage administered to a patient is between 0.001 mg/kg and 15 mg/kg, 0.005 mg/kg and 10 mg/kg, 0.01 mg/kg and 5 mg/kg, 0.001 mg/kg and 4 mg/kg, 0.005 mg/kg and 3 mg/kg, 0.01 mg/kg and 2 mg/kg, 0.001 mg/kg and 1 mg/kg, 0.005 mg/kg and 0.5 mg/kg, 0.010 mg/kg and 0.2 mg/kg, 0.005 mg/kg and 0.050 mg/kg of the patient's body weight.

Exemplary doses of neuregulin also include unit (U) or unit amounts of neuregulin per kilogram of subject or sample weight (e.g., about 1 U per kilogram to about 5000 U per kilogram, about 10 U micrograms per kilogram to about 1000 per kilogram, or about 100 U per kilogram to about 500 U per kilogram). For extened release of neuregulin used in the invention, the dosage administered to a patient is typically 10 U/kg to 1000 U/kg of the patient's body weight, based on weight of the active peptide. Preferably, the dosage administered to a patient is between 1 U/kg and 10,000U/kg, 1 U/kg and 5000 U/kg, 10 U/kg and 5000 U/kg, 10 U/kg and 1000 U/kg, 50 U/kg and 2000 U/kg, 50 U/kg and 1000/kg, 50 U/kg and 500 U/kg, 100 U/kg and 1000 U/kg, 100 U/kg and 500 U/kg, 100 U/kg and 200 U/kg, of the patient's body weight.

In general, the recommended daily dose range of neuregulin in the methods of the invention for the conditions described herein lie within the range of from about 0.001 mg to about 1000 mg per day. Specifically, a total daily dose range should be between 0.001 mg per day and 15 mg per day, 0.005 mg per day and 10 mg per day, 0.01 mg per day and 5 mg per day, 0.001 mg per day and 4 mg per day, 0.005 mg per day and 3 mg per day, 0.01 mg per day and 2 mg per day, 0.001 mg per day and 1 mg per day, 0.005 mg per day and 0.5 mg per day, 0.010 mg per day and 0.2 mg per day. In managing the patient, the therapy can be initiated at a lower dose, perhaps about 0.1 µg to about 1 µg, and increased if necessary up to about 20µg mg to about 1000 µg per day as either a single dose or divided doses, depending on the patient's global response. It may be necessary to use dosages of the active ingredient outside the ranges disclosed herein in some cases, as will be apparent to those of ordinary skill in the art. Furthermore, it is noted that the clinician or treating physician will know how and when to interrupt, adjust, or terminate therapy in conjunction with individual patient response. In certain embodiments, neuregulin is administered in an amount of about 1 U/day to about 10,000 U/day. In some embodiments, it is administered in an amount of about 1 U/day to about 5000 U/day. In some embodiments, it is administered in an amount of about 10U/day to about 2000 U/day. In some embodiments, it is administered in an amount of about 10 U/day to about 1000 U/day. In some embodiments, it is administered in an amount of about 100 U/day to about 200 U/day.

Neuregulin can also be administered in a dosing schedule or "therapeutic cycle." Daily dosage of neuregulin in the therapeutic cycle is described in detail above. The therapeutic cycle can last 2 days, 5 days, 7 days, 10 days, two weeks, three weeks, four weeks, five weeks, or six weeks.

In certaine embodiments, neuregulin is administered daily for each day of the therapeutic cycle. In certain embodiments, neuregulin is administered consecutively for three, four, five, six, seven, eight, nine, ten, eleven or twelve days in a therapeutic cycle.

In certain embodiments, in a therapeutic cycle neuregulin is administered on day 1 of the cycle and the cycle concludes with one or more days of no neuregulin administration In some embodiments, neuregulin is administered daily for 3, 5, 7, or 10 days followed by a resting period in a therapeutic cycle.

### 5.3.1 Combinational Therapy

In one embodiment, the present invention is useful in preventing heart failure and cardiomyopathy in patients being treated with a drug that causes cardiac hypertrophy or heart failure, e.g., fludrocortisone acetate or herceptin. NRG may be administered prior to, simultaneously with, or subsequent to a drug which causes such cardiac diseases.

In another embodiment of the invention, NRG is administered in combination with an effective amount of a compound that acts to suppress a different hypertrophy induction pathway than NRG. In an alternative embodiment, NRG is administered with such hypertrophy suppressors and/or additional components, without limitation, a cardiotrophic inhibitor such as a Ct-1 (cardiotrophin-1) antagonist, an ACE inhibitor, such as captopril (Capoten®), and/or human growth hormone and/or IGF-I (Insulin like growth factor I) in the case of congestive heart failure, or with another anti-hypertrophic, myocardiotrophic factor, anti-arrhythmic, or inotropic factor in the case of other types of heart failure or cardiac disorder.

In another embodiment of the invention, NRG is administered in combination with current therapeutic approaches for treatment of heart failure, including, without limitation, ACE inhibitors and other vasodilators, diuretics, digitalis preparations, beta blockers, blood thinners, angiotensin II receptor blockers, calcium channel blockers or potassium.

ACE inhibitors, which prevent the conversion of angiotensin I to angiotensin II, are vasodilators that cause the blood vessels to expand, lowering the blood pressure and reducing the heart's workload. Vasodilators suitable for use in embodiments of the present invention include, without limitation, the following drugs: quinapril (Accupril®), ramipril (Altace®), captopril (Capoten®), benazepril (Lotensin®), fosinopril (Monopril®), lisinopril (Prinivil® or Zestril®), enalapril (Vasotec®), moexipril (Univasc®), trandolapril, and perindopril. Additional vasodilators useful in the present invention, include, without limitation, isosorbide dinitrate (Isordil®), nesiritide (Natrecor®), hydralazine (Apresoline®), nitrates and minoxidil.

Diuretics cause the kidneys to remove sodium and water from the blood stream, reducing the heart's workload, and include, without limitation, the following drugs: hydrochlorothiazide (HydroDIURIL®), chlorothiazide (Diuril®), furosemide (Lasix®), bumetanide (Bumex®), spironolactone (Aldactone®), triamterene (Dyrenium®), metolazone (Zaroxolyn®), torsemide, indapamide, polythiazide, amiloride, and combination agents (Dyazide®).

Digitalis preparations increase the force of the heart's contractions and include, without limitation, digoxin (Lanoxin®) and digitoxin.

Beta blockers reduce the heart's tendency to beat faster and include, without limitation, the following drugs: carvedilol (Coreg®) metoprolol (Lopressor® or Toprol XL®, atenolol, bisoprolol, labetalol, propranolol, sotalol, pindolol, penbutolol, acebutolol, timolol, nadolol, and betaxolol.

Blood thinners for use in embodiments of the present invention, include, without limitation, warfarin (Coumadin®) and heparin.

Embodiments of the present invention may also use angiotensin II receptor blockers, which, rather than lowering the levels of angiotensin II (as ACE inhibitors do), prevents angiotensin II from effecting the heart and blood vessels. Angiotensin II receptor blockers suitable for use in the present invention, include, without limitation, iosartan (Cozaar®), valsartan (Diovan®), irbesartan (Avapro®), candesartan, eprosartan, telmisartan, and olmesartan.

Calcium channel blockers are generally used to treat high blood pressure often associated with heart failure. Calcium channel blockers suitable for use in the present invention include, without limitation, amlodipine (Norvasc®).

In alternative embodiments of the present invention, NRG can also be combined with the administration of drug therapies for the treatment of heart diseases such as hypertension. For example, NRG can be administered with endothelin receptor antagonists, such as antibodies to the endothelin receptor, and peptides or other such small molecule antagonists; 3-adrenoreceptor antagonists such as carvedilol; x,-adrenoreceptor antagonists; anti-oxidants; compounds having multiple activities (e.g., 3-blocker/a-blocker/anti-oxidant); carvedilol-like compounds or combinations of compounds providing multiple functions found in carvedilol; growth hormone, etc.

Neuregulin agonists alone or in combination with other hypertrophy suppressor pathway agonists or with molecules that antagonize known hypertrophy induction pathways, are useful as drugs for in vivo treatment of mammals experiencing heart failure, so as to prevent or lessen heart failure effects.

Therapeutic formulations of agonist(s) for treating heart disorders are prepared for storage by mixing the agonist(s) having the desired degree of purity with optional physiologically acceptable carriers, excipients, or stabilizers (Remington's Pharmaceutical Sciences, 16th edition, Oslo, A., Ed., 1980), in the form of lyophilized cake or aqueous solutions. Acceptable carriers, excipients, or stabilizers are non-toxic to recipients at the dosages and concentrations employed, and include buffers such as phosphate, citrate, and other organic acids; antioxidants including ascorbic acid; low molecular weight (less than about 10 residues) polypeptides; proteins, such as serum albumin, gelatin, or immunoglobins; hydrophilic polymers such as olyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counter ions such as sodium; and/or non-ionic surfactants such as Tween, Pluronics, or polyethylene glycol (PEG). The antagonist(s) are also suitably linked to one of a variety of nonproteinaceous polymers, e.g., polyethylene glycol, polypropylene glycol, or polyalkylenes, in the manner set forth in US Patent Nos. 4,640,835; 4,496,689; 4,301,144; 4,670,417; 4,791,192 or 4,179,337. The amount of carrier used in a formulation may range from about 1 to 99%, preferably from about 80 to 99%, optimally between 90 and 99% by weight.

The agonist(s) to be used for in vivo administration should be sterile. This is readily accomplished by methods known in the art, for example, by filtration through sterile filtration membranes, prior to or following lyophilization and reconstitution. The agonist(s) ordinarily will be stored in lyophilized form or in solution.

Therapeutic agonist compositions generally are placed into a container having a sterile access port, for example, an intravenous solution bag or vial having a stopper pierceable by a hypodermic injection needle. The agonist(s) administration is in a chronic fashion only, for example, one of the following routes: injection or infusion by intravenous, intraperitoneal, intracerebral, intramuscular, intraocular, intraarterial, or intralesional routes, orally or using sustained-release systems as noted above.

As discussed above, suitable examples of sustained-release preparations include semipermeable matrices of solid hydrophobic polymers containing the protein, which matrices are in form of shaped articles, e.g., films, or microcapsules, Examples of sustained-release matrices include polyesters, hydrogels (e.g., poly(2-hydroxyethylmethacrylate) as described by Langer et al. (1981) J. Biomed. Mater. Res. 15: 167-277 and Langer (1982) Chem. Tech. 12: 98-105, or poly(vinyl alcohol)), polylactides (US Patent No 3,773,919, EP 58,481), copolymers of L-glutamic acid and gamma ethyl-L- . glutamate (Sidman et al. (1983) Biopolymers 22: 547-556), non-degradable ethylene-vinyl acetate (Langer et al. (1981) *supra*) degradable lactic acidglycolic acid copolymers such as the Lupron DepotTM (injectable microspheres composed of lactic acid-glycolic acid copolymer and leuprolide acetate), and poly-D-(-)-3-hydroxybutyric acid (EP 133,988).

The agonist(s) also may be entrapped in microcapsules prepared, for example, by coacervation techniques or by interfacial polymerization (for example, hydroxymethylcellulose or gelatin-microcapsules and poly[methylmethacylate] microcapsules, respectively), in colloidal drug delivery systems (for example, liposomes, albumin microspheres, microemulsions, nano-particles and nanocapsules), or in macroemulsions. Such techniques are disclosed in Remington's Pharmaceutical Sciences, *supra.*

While polymers such as ethylene-vinyl acetate and lactic acid-glycolic acid enable release of molecules for over 100 days, certain hydrogels release molecules for shorter time periods. When encapsulated molecules remain in the body for a long time, they may denature or aggregate as a result of exposure to moisture at 37°C, resulting in a loss of biological activity and possible changes in immunogenicity. Rational strategies can be devised for stabilization depending on the mechanism involved, e.g., using appropriate additives, and developing specific polymer matrix compositions.

Sustained-release agonist(s) compositions also include liposomally entrapped agonists(s). Liposomes containing agonists(s) are prepared by methods known in the art, for example, those disclosed in DE 3,218,121; Epstein et al. (1985) Proc. Natl. Acad. Sci. USA 82: 3688-3692; Hwang et al. (1980) Proc. Natl. Acad. Sci. USA 77:4030-4034: EP 52,322; EP 36,676; EP 88,046; EP 143,949; EP 142,641; Japanese patent application 83-118008; US Patent Nos. 4,485,045 and 4,544,545; and EP 102, 324. A specific example of suitable sustained-release formulation is in EP 647,449.

In another embodiment of the present invention, NRG is combined with or administered in concert with other agents for treating congestive heart failure, including ACE inhibitors (as discussed above), CT-1 inhibitors, human growth hormone, and/or IGF-I. The effective amounts of such agents, if employed will be at the clinician's discretion. Dosage administration and adjustment are determined by methods known to those skilled in the art to achieve the best management of congestive heart failure and ideally takes into account use of diuretics or digitalis, and conditions such as hypotension and renal impairment. The dose will additionally depend on such factors as the type of drug used and the specific patient being treated. Typically the amount employed will be the same dose as that used if the drug were to be administered without agonist; however, lower doses may be employed depending on such factors as the presence of side-effects the condition being treated, the type of patient, and the type of agonists and drug, provided the total amount of agents provides an effective dose for the condition being treated.

It will be appreciated by persons skilled in the art that numerous variations and/or modifications may be made to the invention as shown in the specific embodiments without departing from the spirit or scope of the invention as broadly described. The present embodiments are, therefore, to be considered in all respects as illustrative and not restrictive.

### 5.4 Kits

The invention also provides kits for preverving, perfusion or reperfusion of an organ, such as heart, for transplantation. Such kits comprise in one or more containers the compositions of the present invention, alone or in combination with other materials for heart transplantation. Instructions are optionally included for use the compositions by a physician or by the patient.

### 6. EXAMPLES

The invention will be further illustrated by reference to the following non-limiting Examples. The examples are put forth so as to provide those of ordinary skill in the art with a complete disclosure and description of how to make and use the present invention, and are not intended to limit the scope of what the inventors regard as their invention nor are they intended to represent that the experiments below are all or the only experiments performed. Efforts have been made to ensure accuracy with respect to numbers used but some experimental errors and deviations should be accounted for.

### 6.1 Experimental Methods:

### 6.1.1 Rat left ventricle coronary artery ligation and echocardiography

Wistar male rats (Shanghai Animal Center of Chinese Academy of Science), which weighed 200 ± 20g, were anesthetized by intraperitoneally injecting 100mg/kg (drug/body weight) of ketamine. The neck and chest were depilated and sanitized. An incision was made in the middle front neck to expose the tracheae. An 18G catheter ovemeedle was inserted into the tracheae between the 3rd and 5th cartilage of tracheae. After the needle was drawn out, a plastic cannula was pushed into the trachea 1-2 cm and fixed to connect the Rodent Ventilator (SAR-830/P ventilator - Inspiratory flow rate, 1ml/100g/breath; Respiratory rate, 60 breaths/min). Another incision was made on the left front chest. The skin was blunt dissected to expose the fourth and fifth rib, then the fourth rib was cut by elbowed mosquito forceps. The ventilator (as described above) was linked to the cannula and turned on, and the heart was exposed to check the status of lung and heart. The pericardium was rived off to identify the left atria and the pulmonary arterious cone after the heart was exteriorized through the incision. The left ventricle anterior descending coronary artery between them was ligated tight with 6/0 medical suture before the heart was replaced into the thorax. The thoracic wall was stitched. The ventilator was blocked to full fill the lung. The chest muscle and skin was stitched after the air in the thoracic cavity was gently squeezed out. The ventilators were removed from the rats until constant spontaneous respiration resumed.

The cardiac function of the rats was then examined by echocardiography (Philips Sonos 7500 S4 probe) on the 14th day after ligation. The rats with ejection fraction (hereinafter "EF") values from 30 to 50 percent were separated and grouped (15 rats per group).

### 6.1.2 B. Treating the ligated rats with neuregulin.

The rats were weighed on the 15th day after left ventricle coronary ligation to determine the amount of NRG needed. Rats in the vehicle group received 0.4m1/100g (volume/body weight) of vehicle by IV injection. The vehicle was injected once a day for five days, stopped for two days, and then injected for another five days.

NRG having the sequence of SEQ ID NO: 1 is used: Ser His Leu Val Lys Cys Ala Glu Lys Glu Lys Thr Phe Cys Val Asn Gly Gly Glu Cys Phe Met Val Lys Asp Leu Ser Asn Pro Ser Arg Tyr Leu Cys Lys Cys Pro Asn Glu Phe Thr Gly Asp Arg Cys Gln Asn Tyr Val Met Ala Ser Phe Tyr Lys Ala Glu Glu Leu Tyr Gln.

As discussed further below, the NRG group had osmotic pumps (ALZET osmotic pump 2ML1) implanted on the fifth day after grouping. Each pump contained 2 ml of NRG solution (62.5µg NRG), which contained 933.1U of NRG (as a rat now weighed about 250g) and the infusion speed was about 18.7U/kg/h.

After 7 days, cardiac function of all rats was checked again by echocardiography (Philips Sonos 7500 S4 probe). The next day, hemodynamic parameter check and anatomy check were also undertaken to further confirm the cardiac function of the rats.

**Transplantation of osmotic pump into rats**

The following steps are sterile. 1ml of sterile water and 1ml of sterile 0.9% saline was injected into a vial of NRG (993.1 U, 62.5µg) in the hood successively. The NRG solution was drawn into a sterile syringe. A blunt-tipped needle was exchanged for the syringe and the bubble in the syringe was removed. The pump was held upright and the needle was inserted through the small opening at the top of the upright pump until it could go no further. The plunger was pushed slowly to add NRG solution into the pump until the solution began to overflow the pump. The needle was removed and the pump was wiped clean. The transparent cap of the flow moderator was taken off to expose a short stainless steel tube. The steel tube was then inserted into one end of a 5cm PE60 tube. The syringe needle was inserted into another end of the PE60 tube. The plunger of syringe was pushed to add NRG solution to the flow moderator until it was full. The long tube of the flow moderator was then inserted into the pump until its white flange attached to the pump. The needle was drawn out of the flow moderator before soaking the pump in sterile 0.9% saline at 37°C overnight.

The rats were anesthetized by Ketamine (as described above). The area between neck and shoulder of the rats was depilated and sanitized. The body was covered with a piece of sterile wet cloth. An incision was then carefully made in the skin between the scapulae to locate and separate the external jugular vein. The distal end of the vein from the heart was ligated. A small hole was made by eye scissors on the wall of the external jugular vein and enlarged by microforceps. The PE60 tube connected to the osmotic pump was inserted 2cm into the vein through the hole. The proximal end of the vein from the heart was then bound with PE60 tube to fix the tube. The distal end of the vein surrounding the PE60 tube was tied tight to further fix the tube. Using a hemostat, a tunnel was formed by blunt separation of the skin from the incision to scapula. A pocket was finally made on the back of the rat in the midscapular region by spreading the skin further. The pump was slid through the tunnel into the pocket with the flow moderator pointing away from the incision. The skin incision was then closed with a suture. The rats were put back into the animal room after revival and were fed as usual.

### 6.1.3 Isolation of cardiomyocytes from left ventricle and examination of its contractile speed

Rats are anesthetized with Ketamine. Hearts are quickly excised, rinsed and the ascending aorta cannulated. Continuous retrograde perfusion is initiated on the hearts at a perfusion pressure of 60 cm H₂O. Hearts are first perfused with a nominally Ca²⁺ free modified Krebs solution of the following composition: 110 mM NaCl, 2.6 mM KCl, 1.2 mM KH₂PO₄, 1.2 mM MgSO₄, 2.1 mM NaHCO₃, 11 mM glucose and 4 mM Hepes. This medium is not recirculated and is continually gassed with O₂. After approximately 3 minutes the heart is perfused with modified Krebs buffer supplemented with 3.3% collagenase (Boehringer Mannheim) and trypsin (sigma) until the heart becomes sufficiently blanched and soft. The heart is removed from the cannulae, the atria and vessels discarded and the ventricles are cut into small pieces. The myocytes are dispersed by gentle agitation of the ventricular tissue in fresh collagenase containing Krebs prior to being gently forced through a 200 µm nylon mesh in a tube. The resulting myocytes are resuspended in modified Krebs solution containing 25 µm calcium. Myocytes are made calcium tolerant by addition of a calcium solution (100 mM stock) at 10 minute intervals until 100 µM calcium is achieved. After 30 minutes the supernatant is discarded and 30-50 ml of Tyrode buffer (137 mM NaCl, 3.7 mM KCl, 0.5 mM MgCl, 11 mM glucose, 4 mM Hepes, and 1.2 mM CaCl₂, pH 7.4) is added to cells. Cells are kept for 60 min at 37°C prior to initiating experiments and used within 5 hrs ofisolation.

Aliquots of Tyrode buffer containing myocytes are placed in perfusion chambers complete with heating platforms. Myocytes are allowed to attach, the chambers heated to 37°C, and the cells then perfused with 37°C Tyrode buffer. Myocytes are field stimulated at 0.5Hz in with platinum electrodes. Only cells that have clear striations, and are quiescent prior to pacing are used for contractility experiments. To determine basal contractility, myocytes are imaged through a 40x objective and using a variable frame rate (60-240 Hz) charge-coupled device camera, the images are digitized and displayed on a computer screen at a sampling speed of 240 Hz. [Frame grabber, myopacer, acquisition, and analysis software for cell contractility are available from IonOptix (Milton, Ma.).] Using edge detection strategy, contractility of the myocytes and contraction and relaxation velocities are continuously recorded.

### 6.2 Example 2: The Effect of Neuregulin on the Relaxation of Heart

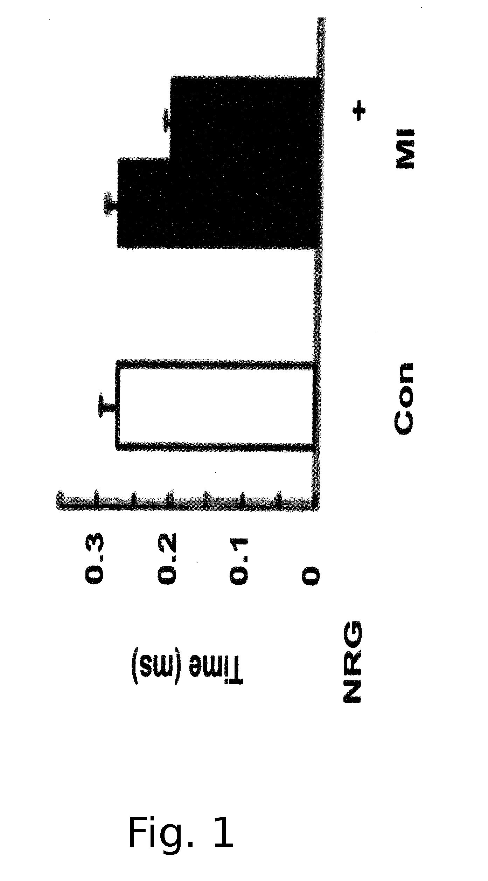

Normal rats were separated into 3 groups (15 rats per group). One group was sham operated as control. For another two groups of rats, left ventricle coronary artery was ligated as described in methods to form myocardial infarcted rats (MI rats). MI rats were further grouped into 2 groups (15 rats per group). One group of rats were treated with vehicle, the other group of rats were treated with neuregulin by osmotic pump. After the treatment, hearts were taken from all the rats and cardiomyocytes were isolated. The contractile index of cardiac myocytes was measured. As shown in Fig 1, compare with myocytes from MI rats treated with vehicle, myocytes from MI rats treated with neuregulin relax much faster. This shows that neuregulin can have beneficial effect on the heart's relaxation.

### 6.3 Example 3: The effect of Neuregulin on the Preservation of Heart for Transplantation

Hearts were collected from normal rat and divided into 3 groups (7 hearts in each group). Cardiac function was examined by echocardiography while the hearts were perfused with Krebs solution. Then one group was stored in Celsior solution for 6 hours at 2°C, the second group was stored in Celsior solution containing l4nM NRG for 6 hours at 2°C, the third group was treated with 0.1 µM wortmannin for 15 min before stored in Celsior solution containing 14nM NRG for another 6 hours at 2°C. After storage, hearts were perfused with Krebs solution again and their cardiac function was measured by echocardiography. Some heart tissue was collected and homogenized for Western blot to detect AKT phosphorylation.

The results on cardiac function were shown in table 1.

**Table 1, Cardiac function of rat hearts after 6 hours' storage**

| group | Aortic flow (%recovery) | Coronary flow (% recovery) | Cardiac output (% recovery) | Heart rate (% recovery) |
|---|---|---|---|---|
| Celsior | 1.2±0.4 | 1.7±1.1 | 1.4±0.6 | 32.2±8.6 |
| Celsior with NRG | 15±7.2 | 39±11.5 | 21.2±7.9 | 75.7±11.7 |
| Wortmannin(pre-treated) Celsior with NRG | 1.28±0.7 | 0.79±0.7 | 1.17±0.7 | 42.9±7.1 |

| | | | | |
|---|---|---|---|---|
| The value in the table was expressed as percentage of cardiac index measured right after heart collection from rats. | | | | |

As shown in Table 1, hearts preserved in Celsior with neuregulin have much higher cardiac output (15.14 times) and coronary flow (22.94 times) than hearts preserved in Celsior alone. This shows that neuregulin can better preservation of heart before transplantation. It indicates that neuregulin can be added to Celsior to preserve the cardiac function of heart better prior to transplantation.

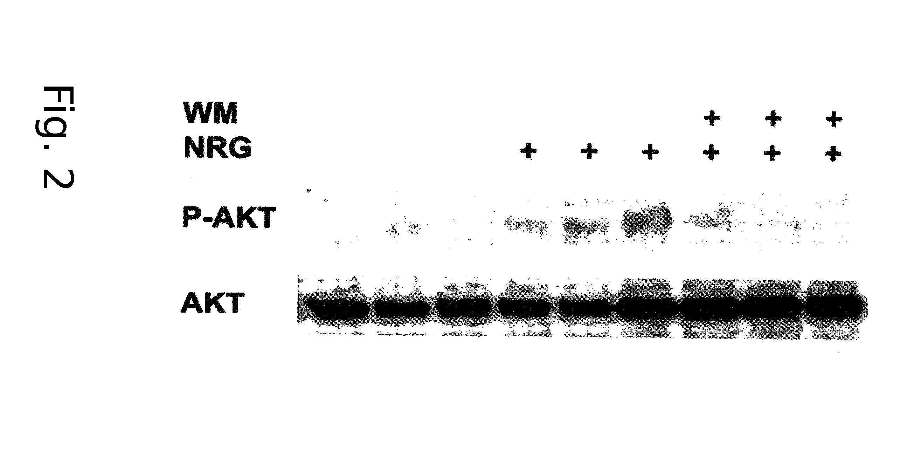

Table 1 also shows that after preincubation with wortmannin, neuregulin's effect on heart was completely blocked. Fig 2 shows that preincubation with wortmannin almost fully inhibited AKT phosphorylation in heart tissue. It suggested that wortmannin inhibited neuregulin's effect on heart through blocking AKT signaling pathway in the heart, this indicated that neuregulin's effect on isolated heart should be attributed to its downstream AKT signaling.

### 6.4 Example 4: Use of Neuregulin to Treat Stable Chronic Heart Failure

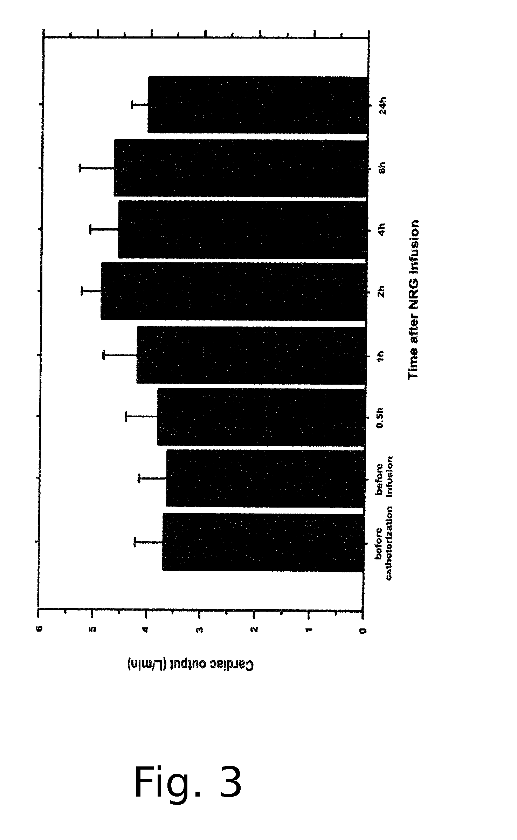

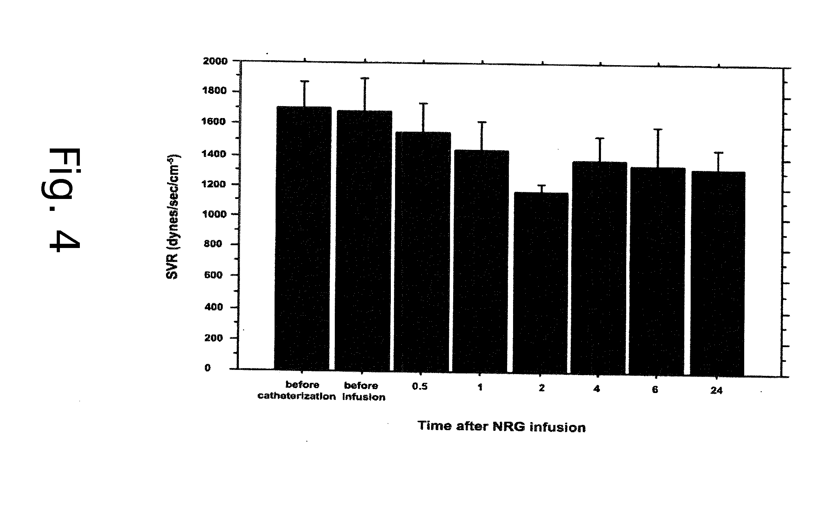

6 patients with stable chronic heart failure were treated with continuous intravenous infusion of neuregulin at 0.2µg/kg/hour (drug weight/body weight/hour) for 6 hours. A baseline right heart catheterisation was performed and Swan Ganz catheter left in situ to monitor haemodynamics for 24 hours. The results were shown in Fig 3 and Fig 4.

As shown in Fig 3, the average cardiac output of patients increased 29.7% from 3.7±1.0 (baseline) to 4.8±0.8 L/min at 2 hours after infusion. And Fig 4 shows that the systemic vascular resistance (SVR) fell by 29% from 1679.3±336.3 to 1199.5±127.6 dynes-see-cm⁻⁵. The results showed that NRG acutely improves cardiac output and lowers SVR in patients with stable chronic heart failure.

The scope of the invention is not limited by the description of the examples. Modifications and alterations of the present invention will be apparent to those skilled in the art without departing from the scope and spirit of the present invention. Therefore, it will be appreciated that the scope of this invention is to be defined by the appended claims, rather than by the specific examples which have been presented by way of example.
The application relates *inter alia* to the following embodiments:
1. A composition for perfusion, preservation or reperfusion of an organ for transplantation, comprising a preservation solution and an effective amount of neuregulin.
2. The composition of embodiment 1, wherein the preservation solution is selected from the group consisting of Celsior Solution, Krebs-Henseleit solution, normal saline solution, University of Wisconsin solution, St. Thomas II solution, Collins solution, and Stanford solution.
3. The composition of embodiment 1, wherein the preservation solution is Celsior solution.
4. The composition of embodiment 1, wherein the preservation solution is Krebs-Henseleit solution.
5. The composition of embodiment 1, wherein neuregulin comprises the amino acid sequence of SEQ ID NO:1.
6. The composition of embodiment 1, wherein neuregulin is at the concentration of about 10-20nM.
7. The composition of embodiment 1, wherein neuregulin is at the concentration of about 14 nM.
8. A method for preserving an organ for transplantation, said method comprising contacting the organ with an effective amount of neuregulin.
9. The method of embodiment 8, wherein the organ is heart.
10. The method of embodiment 8, wherein neuregulin comprises the amino acid sequence of SEQ ID NO: 1.
11. The method of embodiment 8, wherein neuregulin is administered in an amount sufficient to activate AKT signaling pathway.
12. A method for increasing the relaxation speed of heart in a subject, said method comprising administering an effective amount of neuregulin to a subject.
13. The method of embodiment 12, wherein the subject is human.
14. A method for treating chronic heart failure in a subject, comprising administering an effective amount of neuregulin for six hours to a subject in need thereof.
15. The method of embodiment 14, wherein the subject is human.
16. The method of embodiment 14, wherein neuregulin improve cardiac output in the subject.
17. The method of embodiment 14, wherein neuregulin lower systemic vascular resistance in the subject.

## Claims

1. A composition for perfusion, preservation or reperfusion of an organ for transplantation, comprising a preservation solution and an effective amount of neuregulin.

2. The composition of Claim 1, wherein the preservation solution is selected from the group consisting of Celsior solution, Krebs-Henseleit solution, normal saline solution, University of Wisconsin solution, St. Thomas II solution, Collins solution, and Stanford solution.

3. The composition of Claim 1, wherein neuregulin comprises the amino acid sequence of SEQ ID NO:1.

4. The composition of Claim 1, wherein neuregulin is at the concentration of about 10- 20 nM.

5. The composition of Claim 1, wherein neuregulin is at the concentration of about 14 nM.

6. A method for preserving an organ for transplantation, said method comprising contacting the organ with an effective amount of neuregulin.

7. The method of Claim 6, wherein the organ is heart.

8. The method of Claim 6, wherein neuregulin comprises the amino acid sequence of SEQ ID NO:1.

9. The method of Claim 6, wherein neuregulin is administered in an amount sufficient to activate AKT signaling pathway.

10. Neuregulin for use in a method for increasing the relaxation speed of heart in a subject, said method comprising administering an effective amount of neuregulin to a subject.

11. Neuregulin for use of Claim 10, wherein the subject is human.

12. Neuregulin for use in a method for treating chronic heart failure in a subject, said method comprising administering an effective amount of neuregulin for six hours to a subject in need thereof.

13. Neuregulin for use of Claim 12, wherein the subject is human.

14. Neuregulin for use of Claim 12, wherein neuregulin improves cardiac output in the subj ect.

15. Neuregulin for use of Claim 12, wherein neuregulin lowers systemic vascular resistance in the subj ect.
